# EUROPEAN PATENT APPLICATION

(11) **EP 4 589 294 A1**
(43) Date of publication of application: **23.07.2025**
(21) Application number: 24222178.6
(22) Date of filing: 20.12.2024
(51) Int. Cl.: G01N 33/00, G01N 1/28

(54) **SYSTEMS, APPARATUSES, AND METHODS FOR TESTING GAS SENSORS**

(30) Priority: 22.01.2024 IN 202411004303
(71) Applicant: Honeywell International Inc., Charlotte, NC 28202 (US)
(72) Inventor: SHANBHOGUE, Rachan, Charlotte, 28202 (US); KUMAR, Nirmal A., Charlotte, 28202 (US); JAYAKUMAR, Prince Ashwin Kumar Anburaj, Charlotte, 28202 (US)
(74) Representative: Haseltine Lake Kempner LLP

(57) **Abstract**

Systems, apparatuses, and methods for testing a gas sensor are provided. A sensor test system may include a metal box that includes a first inlet and configured to enclose a gas sensor configured to sense a presence of a gas of interest. The system may include a liquid gas bubbler configured to receive a carrier gas and generate the gas of interest from a liquid form of the gas of interest using the carrier gas. The system may include a first flow controller configured to control a flow rate of the carrier gas to the liquid gas bubbler and the gas of interest from the liquid gas bubbler to the first inlet of the metal box. The system may include a thermal chamber configured to enclose the liquid gas bubbler and the metal box and configured to set a temperature inside the thermal chamber to a desired temperature.

## Description

### TECHNICAL FIELD

Embodiments of the present disclosure are generally directed to systems, apparatuses, and methods for testing one or more sensors. In particular, some embodiments of the present disclosure relate to systems, apparatuses, and methods for testing one or more gas sensors.

### BACKGROUND

Various substances may emit gasses such as through off-gassing. A gas sensor may be used to detect the emitted gas. Gas sensors may need to be tested, for example calibrated and/or characterized before they are used for sensing the mitted gas.

Applicant has identified many technical challenges and difficulties associated with testing gas sensors. Through applied effort, ingenuity, and innovation, Applicant has solved problems related to testing gas sensors by developing solutions embodied in the present disclosure, which are described in detail below.

### BRIEF SUMMARY

Various embodiments described herein relate to systems, apparatuses, products, and methods for testing, calibrating, and/or characterizing a gas sensor.

In various embodiments, a sensor test system is provided. The sensor test system may include a metal box comprising a first inlet, wherein the metal box is configured to enclose a gas sensor configured to sense a presence of a gas of interest; a liquid gas bubbler configured to receive a carrier gas and generate the gas of interest from a liquid form of the gas of interest using the carrier gas; a first flow controller configured to control a flow rate of the carrier gas to the liquid gas bubbler and the gas of interest from the liquid gas bubbler to the first inlet of the metal box; and a thermal chamber configured to enclose the liquid gas bubbler and the metal box, and configured to set a temperature inside the thermal chamber to a desired temperature.

In various embodiments, the sensor test system further comprising a gas channel configured to direct the gas of interest from the first inlet of the metal box to the gas sensor; a carrier gas container configured to: provide the carrier gas to the liquid gas bubbler, wherein the first flow controller is placed between the carrier gas container and the liquid gas bubbler; and provide carrier gas to the metal box through a second inlet of the metal box; and a second flow controller placed between the carrier gas container and the second inlet of the metal box and configured to control a flow rate of the carrier gas to the metal box.

In various embodiments, the sensor test system further comprising a water bubbler configured to provide water vapor to the metal box through a third inlet of the metal box; and a third flow controller placed between the water bubbler and the third inlet and configured to control a flow rate of water vapor to the metal box.

In various embodiments, the sensor test system further comprising a heater configured to increase a temperature of water in the water bubbler and facilitate generating the water vapor. In various embodiments, the water bubbler comprises a metal coin configured to rotate at a high speed and to cause the water to vaporize; and the heater is a magnetic heater and is configured to cause the coin to rotate and increase a temperature of the coin to facilitate generating the water vapor.

In various embodiments, the metal box is configured to contain a temperature sensor and a humidity sensor, wherein the temperature sensor is configured to measure a temperature of the inside of the metal box and the humidity sensor is configured to measure a humidity inside the metal box.

In various embodiments, the metal box comprises a first outlet configured to pass the gas of interest, the carrier gas, and the water vapor to an external environment.

In various embodiments, the sensor test system further comprising a scrubber coupled to the first outlet, the scrubber configured to remove hazardous substance from the gas of interest, the carrier gas, and the water vapor before flowing to the external environment.

In various embodiments, the sensor test system further comprising a vacuum pump coupled to a second outlet of the metal box, wherein the vacuum pump configured to purge the gas of interest from the metal box in combination with a flow of the carrier gas through the metal box; and a condensation trap comprising an ambient condenser or an active condenser, wherein the condensation trap is coupled to the second outlet of the metal box and placed outside the thermal chamber and is configured to convert the gas of interest to the liquid form of the gas of interest; and a liquid gas channel coupled to the condensation trap and the liquid gas bubbler, the liquid gas channel configured to direct the liquid form of the gas of interest to the gas bubbler.

In various embodiments, the liquid gas bubbler comprises a sintered tube configured to break large bubbles generated by the carrier gas to finer bubbles and facilitate generating the gas of interest.

Various embodiments provide a method for testing a gas sensor, the method comprising generating, using a liquid gas bubbler, a gas of interest from a liquid form of the gas of interest; controlling, using a first flow controller, a flow rate of a carrier gas to the liquid gas bubbler, wherein the flow of the carrier gas to the liquid gas bubbler determines a flow rate of the generated gas of interest; flowing, via a first inlet of a metal box, the gas of interest to the metal box configured to enclose the gas sensor, wherein the gas sensor is configured to sense a presence of the gas of interest; and adjusting, using a thermal chamber, a temperature of the liquid gas bubbler to be approximately the same as a temperature of the metal box.

In various embodiments, the method further comprising converting, using a condensation trap comprising an ambient condenser or an active condenser placed outside the thermal chamber, the gas of interest to the liquid form of the gas of interest; and directing, using a liquid channel coupled to the condensation trap and the liquid gas bubbler, the liquid form of the gas of interest to the gas bubbler, wherein the thermal chamber is configured to enclose the liquid gas bubbler and the metal box.

In various embodiments, the method further comprising directing, via a gas channel, the gas of interest to the gas sensor from the first inlet of the metal box; flowing, via a second inlet of the metal box, the carrier gas to the metal box; and controlling, via a second flow controller, a flow rate of the carrier gas to the metal box, wherein the second flow controller is placed between a carrier gas container and the metal box.

In various embodiments, the method further comprising controlling a concentration of the gas of interest in the metal box by controlling, using the first flow controller, the flow rate of the gas of interest to the metal box; and controlling, using the second flow controller, the flow rate of the carrier gas to the metal box.

In various embodiments, the method further comprising generating, using a water bubbler, water vapor; flowing, using a third inlet of the metal box, the water vapor to the metal box; and controlling, using a third flow controller, a flow rate of water vapor, wherein the third flow controller is placed between the water bubbler and the metal box.

In various embodiments, the method further comprising heating, using a magnetic heater, a metal coin in water contained in the water bubbler; heating, using the metal coin, the water in the water bubbler; and rotating, using the magnetic heater, the metal coin in the water contained in the water bubbler to generate water vapor.

In various embodiments, the method further comprising controlling a humidity inside the metal box by controlling, using the second flow controller, the flow rate of the carrier gas to the metal box; and controlling, using the third flow controller, the flow rate of the water vapor to the metal box.

In various embodiments, the method further comprising measuring, using a humidity sensor in the metal box, a humidity inside the metal box; and controlling the humidity inside the metal box to a desired humidity level using the measured humidity inside the metal box.

In various embodiments, the method further comprising measuring, using a temperature sensor in the metal box, a temperature inside the metal box; and controlling the temperature inside the metal box to a desired temperature level using the measured temperature inside the metal box.

Various embodiments of the present disclosure provide a method comprising converting, using a liquid gas bubbler and a carrier gas, a substance of interest from a liquid form to a gas form; flowing the gas from of the substance of interest to a metal box via a first inlet of the metal box; varying a humidity level inside the metal box by flowing water vapor generated by a water bubbler to the metal box using a second inlet of the metal box; flowing the carrier gas directly to the metal box using a third inlet of the metal box; controlling a flow rate of the gas form of the substance of interest, a flow rate of water vapor, and a flow rate of the carrier gas to the metal box; controlling a temperature of the liquid gas bubbler and the metal box using a thermal chamber enclosing the liquid gas bubbler and the metal box; and purging the substance of interest from the metal box using the carrier gas and a vacuum pump

The above summary is provided merely for purposes of summarizing some example embodiments to provide a basic understanding of some aspects of the disclosure. Accordingly, it will be appreciated that the above-described embodiments are merely examples and should not be construed to narrow the scope or spirit of the disclosure in any way. It will also be appreciated that the scope of the disclosure encompasses many potential embodiments in addition to those here summarized, some of which will be further described below.

### BRIEF DESCRIPTION OF THE SEVERAL VIEWS OF THE DRAWINGS

FIG. 1 is a schematic diagram illustrating various aspects of the subject matter in accordance with the disclosure.
FIG. 2 is a schematic diagram illustrating various aspects of the subject matter in accordance with the disclosure.
FIG. 3 is a schematic diagram illustrating various aspects of the subject matter in accordance with the disclosure.
FIG. 4A is a schematic diagram illustrating a sensor test system in accordance with various embodiments of the present disclosure.
FIG. 4B is a schematic diagram illustrating various aspects of a sensor test system in accordance with various embodiments of the present disclosure.
FIG. 5 is a schematic diagram illustrating a liquid gas bubbler in accordance with various embodiments of the present disclosure.
FIG. 6 is a schematic diagram illustrating a water bubbler in accordance with various embodiments of the present disclosure.
FIG. 7 is a schematic diagram illustrating a controller in accordance with various embodiments of the present disclosure.
FIG. 8 is a flowchart illustrating a method in accordance with various embodiments of the present disclosure.

### DETAILED DESCRIPTION

Embodiments of the present disclosure will now be described more fully herein with reference to the accompanying drawings, in which some, but not all, embodiments of the disclosure are shown. Indeed, various embodiments of the disclosure may be embodied in many different forms and should not be construed as limited to the embodiments set forth herein; rather, these embodiments are provided so that this disclosure will satisfy applicable legal requirements. Like reference numerals refer to like elements throughout.

The phrases "in one embodiment," "according to one embodiment," "in some embodiments," "in various embodiments" and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

Use of broader terms such as "comprises," "includes," and "having" should be understood to provide support for narrower terms such as "consisting of," "consisting essentially of," and "comprised substantially of". Use of the terms "optionally," "may," "might," "possibly," and the like with respect to any element of an embodiment means that the element is not required, or alternatively, the element is required, both alternatives being within the scope of the embodiment(s). Also, references to examples are merely provided for illustrative purposes, and are not intended to be exclusive.

The term "electronically coupled," "electronically coupling," "electronically couple," "in communication with," "in electronic communication with," or "connected" in the present disclosure refers to two or more elements or components being connected through wired means and/or wireless means, such that signals, voltage/current, data and/or information may be transmitted to and/or received from these elements or components.

The term "mechanically coupled" or "coupled" in the present disclosure refers to two or more mechanical elements (for example, but not limited to, a frame, a surface, a support unit, a joint, etc.) being physically connected in various ways such as directly, through intermediary elements, and/or using fastener(s), clasps, clamps, joints, pin j oint, axle, hinge, adhesive, etc. The term "mechanically coupled" may refer to any of movable, turntable, swiveling, pivoting, fixed, and/or stationary, etc. physical connections.

Various embodiments of the present disclosure are directed to improved systems, apparatuses, products, and methods for testing gas sensors. It should be readily appreciated that the embodiments of the systems, apparatus, and methods described herein may be configured in various additional and alternative manners in addition to those expressly described herein.

Detecting the presence of various gases may be desired in various devices, methods, or systems. For example, a gas produced via off-gassing may be an indication of a hazardous condition. Therefore, detecting the produced and/or emitted gas may be used in detecting and/or preventing the hazardous condition.

Various embodiments of the present disclosure provide systems, apparatus, and methods for testing gas sensors. Testing may include calibrating and/or characterizing the gas sensor. For example, calibrating a sensor may include determining an output from the sensor in a controlled environment and/or adjusting various parameters of the sensor. Characterizing a sensor may include determining an output of a sensor in a controlled condition.

Various embodiments of the present disclosure test a sensor configured to detect the presence of a gas of interest. In various embodiments, the gas of interest is generated from a substance of interest. The gas of interest may be generated from a liquid form of the substance of interest for testing the gas sensor.

In example embodiments, the gas of interest (which may in short be referred to as gas) may include any gas from the carbonate gas family, for example including but not limited to propylene carbonate (PC), ethyl methyl carbonate (EMC), dimethyl carbonate (DMC), ethylene carbonate (EC), etc.

In an example, the gas of interest may be generated by a battery. A battery may encounter a condition known as thermal runaway which may result in fire and/or other hazardous conditions. Before thermal runaway occurs, the battery may enter a venting stage where a gas, such as PC is emitted from the battery. PC may be generated due to off-gassing from an electrolyte of the battery for example when it is in an elevated temperature. Gas sensors may be used to detect PC during the venting stage and provide warning and/or alert before thermal runaway occurs. Therefore, gas sensors contribute to battery and user safety where batteries are used, such in an electric vehicle (EV).

Gas sensors may require a test setup to calibrate and/or characterize the gas sensor at various temperatures and/or humidity levels. For example, the various temperatures or humidity levels may be those that the battery pack of an EV may be exposed to. In various embodiments, systems, devices, and methods are provided to expose the gas sensor to the gas of interest in various temperatures and/or humidity levels in order to calibrate and/or characterize the sensor for various operating conditions.

Various embodiments provided by the present disclosure may apply to calibration and/or characterization setup of sensor(s) for detecting any other gas, such as gases generated by off gassing. When used to determine thermal runaway of a battery, various embodiments of the present disclosure may be used for calibration and/or characterization setup of sensor(s) configured to detect any carbonate gas family that may be generated by the battery. Various embodiments of the present disclosure, convert the gas of interest from a liquid form to a gas form for testing.

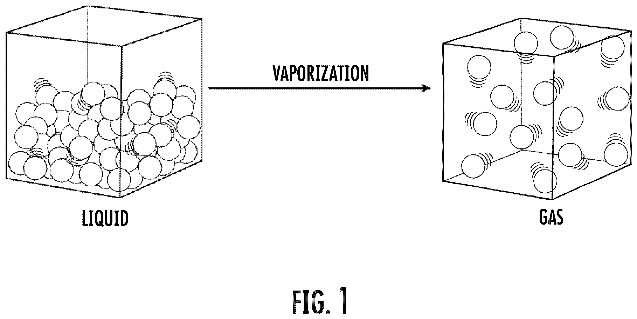

Referring now to FIG. 1 a schematic diagram illustrating a vaporization process is provided in accordance with the present disclosure. Many forms of gasses that need to be detected, may not readily be available in a gas form due to factors like their natural state, reactivity, transportations and storage considerations, and/or safety reasons, etc. Therefore, a gas of interest may be more readily available in a liquid form and be converted to its gas form from a liquid form. Converting liquid to gas may pose a challenge. The conversion may involve the process of vaporization or evaporation to separate the molecules in order to convert a liquid into a gas. For example, PC gas is not readily available in a gas form due to factors described above and a liquid form of PC may be converted into a gas form of PC.

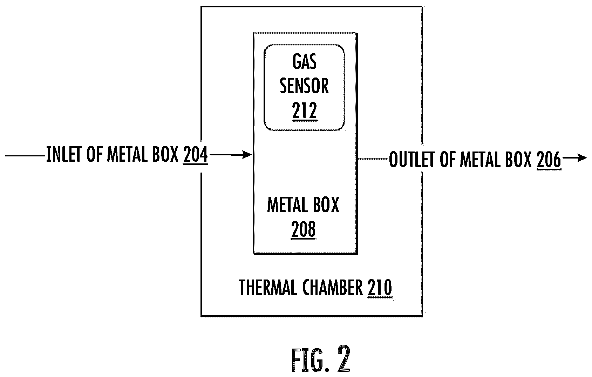

Referring now to FIG. 2 a schematic diagram illustrating a metal box 208 and a thermal chamber 210 is provided in accordance with the present disclosure. A gas sensor 212 may be used to determine a presence of a gas of interest, for example PC. To calibrate and/or characterize gas sensor 212, it may be placed inside the metal box 208 configured to contain gas. The metal box 208 may have an inlet of metal box 204 and an outlet of metal box 206 for gas in-flow and out-flow. The gas sensor 212 may be exposed to the gas after it is converted from a liquid form to a gas form and passed into the metal box 208 via the inlet of metal box 204.

Enclosing the gas sensor 212 in a closed metal box 208 during testing is beneficial when dealing with a gas such as PC, since it can be combustible and breathing it may be hazardous to health. The metal box 208 provides safety by preventing the release or spread of the gas, hence protecting against potential hazards.

Testing the sensor at various temperatures and humidity levels is important to assess its performance and accuracy in different environmental conditions, ensuring its reliability and suitability for diverse operating environments. The gas sensor 212 can be exposed to different temperatures even in a closed environment by placing the metal box 208 inside a thermal chamber 210 and adjusting the thermal chamber's temperature as needed for testing.

However, maintaining specific humidity levels within a sealed metal box during testing may be challenging because the enclosure can restrict the exchange of moisture between the interior and exterior environments.

Additionally, before starting to calibrate and/or characterize a new gas sensor or the same gas sensor with a different temperature and/or humidity condition, the gas of interest may need to be removed from the metal box to examine how the gas sensor functions without the gas present. This process may be referred to as purging. In various approaches that have been used, the metal box may be purged using nitrogen gas (N2) to remove the gas. While both inlet of metal box 204 and outlet of metal box 206 are open, N2 may be passed into the metal box using the inlet of metal box 204, flown through the metal box, and passed out of the metal box through the outlet of metal box 206.

However, the molecular weight of the gas of interest, such as PC gas, may be higher compared to the molecular weight of nitrogen gas. In such conditions, the gas tends to settle at the bottom of the metal box, while the nitrogen gas will occupy the upper space. This can reduce the efficiency of purging. Therefore, purging the metal box from the existing gas may be another challenge in calibrating and/or characterizing the gas sensor 212.

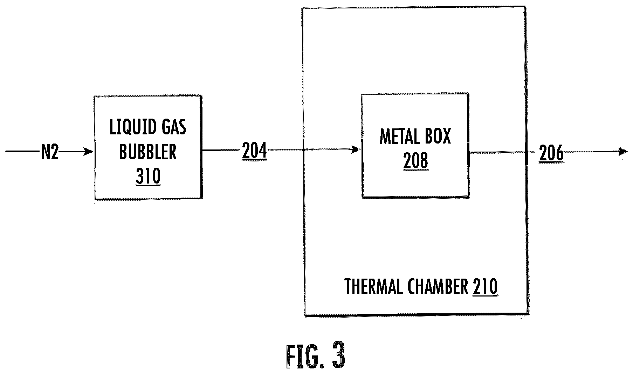

Referring now to FIG. 3 a schematic diagram illustrating a liquid gas bubbler 310 is provided in accordance with various approaches that have been used. The liquid gas bubbler 310 may be used to convert a gas of interest from its liquid form to its gas form before flowing to the metal box 208.

However, the temperature inside the metal box may be similar to the temperature inside the thermal chamber 210 and higher than the temperature outside of the thermal chamber 210. Normally higher temperature increases gas pressure; therefore, gases tend to move from the region of higher temperature to the region of lower temperature. Therefore, in such conditions, the normal flow of the gas may be from the metal box to the liquid gas bubbler. For example, if the temperature of the metal box is at 60°C and the temperature of the liquid gas bubbler 310 containing the liquid form of the gas is at 25°C, it is challenging to cause the gas to flow to the metal box 208 from the liquid gas bubbler 310. This may be because and the pressure of the liquid gas in the liquid gas bubbler 310 may be lower than the gas pressure required to reach the metal box 208, due to temperature difference.

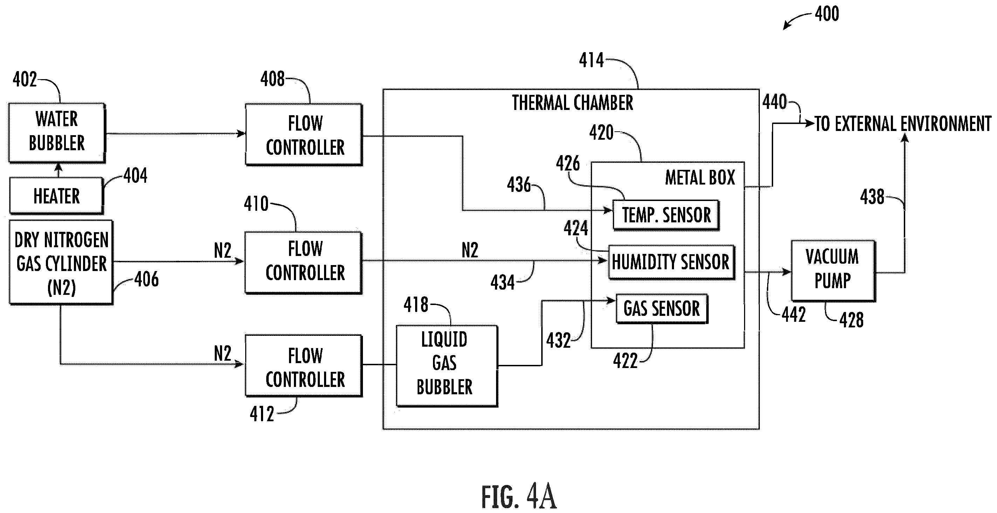

Referring now to FIG. 4A a schematic diagram illustrating a sensor test system 400 is provided in accordance with various embodiments of the present disclosure. The sensor test system 400 overcomes various challenges with testing a gas sensor as previously described.

In various embodiments, the sensor test system 400 includes a carrier gas container configured to contain a carrier gas. For example, the carrier gas container may be a dry nitrogen gas cylinder 406 which is a container that holds compressed nitrogen (N2) gas. In various embodiments, the dry nitrogen gas cylinder 406 is free of moisture.

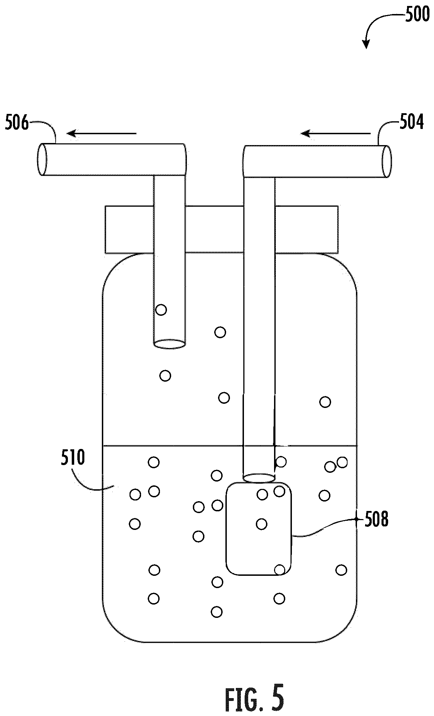

In various embodiments, the N2 generated by the dry nitrogen gas cylinder 406 may flow through a first flow controller 412. A flow controller may be a device configured to control and/or regulate the flow rate of a gas flowing through it. The first flow controller 412 may be configured to control and/or regulate the flow rate of N2 from the dry nitrogen gas cylinder 406 to a liquid gas bubbler 418. The liquid gas bubbler 418 may include a liquid form of the gas of interest and converts it to a gas form to be detected by a gas sensor. A liquid gas bubbler is further illustrated and described below with reference to FIG. 5.

In various embodiments, the sensor test system 400 includes a metal box 420. In various embodiments, the metal box 420 may be a hermetically sealed metal box 420. The metal box 420 may enclose a gas sensor 422, humidity sensor 424, and a temperature sensor 426.

In various embodiments, the gas sensor 422 is configured to sense a presence of the gas of interest. Keeping the gas sensor 422 inside the box may isolate the gas sensor 422 from outside influences and maintain a controlled testing environment.

In various embodiments, the 420 has three inlets and two outlets. In various embodiments, the inlets and outlets of the metal box 420 may be combined into single inlet and/or single outlet. Various flow controlling and/or regulating devices may be used in the single inlet and/or single outlet of the metal box 420.

The gas of interest flows from the liquid gas bubbler 418 to the metal box 420 through a first inlet 432. The output of liquid gas bubbler 418 may include a mix of the carrier gas (e.g., N2) and the gas. By varying the flow of N2 through the liquid gas bubbler 418, an amount of the gas generated and passed to the metal box 420 varies. In various embodiments, using first flow controller 412, a flow of N2 passing though the liquid gas bubbler 418, the amount of gas generated and passed to the metal box 420, and consequently the concentration of the gas in the metal box 420 is determined. An approximately continuous flow of the gas of interest may pass through the metal box 420 from the first inlet 432 to the first outlet 440.

In various embodiments, an approximately constant concentration of the gas of interest in the metal box 420 is maintained using, at least, a flow of N2 set by and/or controlled by the first flow controller 412. For example, by increasing the flow of N2 using the first flow controller 412, a concentration of the gas of interest in metal box 420 may increase and by decreasing a flow of N2 using the first flow controller 412, a concentration of the gas of interest in the metal box 420 may decrease. By increasing a rate of flow of N2 using the first flow controller 412, a higher rate of the gas of interest may be generated in liquid gas bubbler 418 and passed into the metal box 420 and a concentration of the gas in the metal box 420 may increase. By decreasing a rate of flow of N2 using the first flow controller 412, a lower rate of the gas of interest may be generated in liquid gas bubbler 418 and passed into the metal box 420 and a concentration of the gas in the metal box 420 may decrease.

In various embodiments, the concentration of the gas of interest in metal box 420 may be instead and/or additionally controlled using third flow controller 410. In various embodiments, the third flow controller 410 may control a flow of N2 directly to the metal box 420 via third inlet 434. Increasing a flow of N2 to the metal box 420 using third flow controller 410 may decrease the concentration of the gas of interest in metal box 420 and decreasing the flow of N2 to the metal box 420 using third flow controller 410 may increase the concentration of the gas of interest in the metal box 420.

In various embodiments, there is a continuous flow of the gas of interest and/or N2 through the metal box 420. The gas of interest and/or N2 flows in the metal box 420 using the first inlet 432 or third inlet 434 and flow out of the metal box 420 through the first outlet 440 to the external environment. In various embodiments, a scrubber is used at the first outlet 440 to prevent any hazardous material from being dissipated in the external environment.

In various embodiments, the sensor test system 400 includes a thermal chamber 414 configured to enclose liquid gas bubbler 418 and metal box 420. A concentration and/or pressure of the gas of interest in the metal box 420 may additionally or instead be controlled using the temperature set by the thermal chamber.

In various embodiments, by increasing the temperature the pressure inside the metal box 420 may increase, which may lead to more outflow of the gas of interest from the metal box 420 and reduction of the concentration of the gas of interest in metal box 420. Decreasing the temperature may reduce the pressure inside the metal box 420 and increase an inflow of the gas of interest to metal box 420 and increase a concentration of the gas of interest in metal box 420.

In various embodiments, the concentration of the gas of interest in metal box 420 may be fixed and/or dynamically determined using at least one of the methods described herein.

In various embodiments, a temperature sensor 426 and a humidity sensor 424 are also placed inside the metal box to monitor the temperature and humidity. In various embodiments, the gas sensors 422 is calibrated and/or characterized in various temperatures or humidity conditions to simulate the conditions where the gas sensor 422 is deployed and in which the gas of interest may be released and/or may need to be detected.

In various embodiments, the metal box 420 is placed inside a thermal chamber 414. The thermal chamber 414 is configured to vary and/or set a desired temperature inside. The metal box 420 is heat conductive, hence the temperature inside the metal box 420 will be set using the thermal chamber 414.

In various embodiments, a temperature in which the gas sensor 422 is tested in metal box 420 is determined using the thermal chamber 414. Certain required temperatures or a temperature rage for testing gas sensor 422 may be determined based on various condition the gas sensor 422 will be deployed in. In various embodiments, when the temperature sensor 426 determines that a temperature inside the metal box 420 is above a desired temperature, the thermal chamber 414 reduces the temperature inside the thermal chamber 414. When the temperature sensor 426 determines that the temperature inside the metal box 420 is below the desired temperature, the thermal chamber 414 increases the temperature inside the thermal chamber 414.

In various embodiments, by placing the liquid gas bubbler 418 inside the thermal chamber 414, the liquid gas bubbler 418 is placed in an environment with the same temperature as the metal box 420. Therefore, the liquid gas bubbler 418 and the metal box 420 may have approximately the same temperature.

In example embodiments, increasing a temperature of the liquid gas bubbler 418 may also promote evaporation of the liquid form of the gas of interest and increase the gas concentration. In some examples, by having an approximate similar temperature of the liquid gas bubbler 418 and the metal box 420 the gas generated by the liquid gas bubbler 418 flows more easily to the metal box 420. In example embodiments, a higher gas pressure is generated at the liquid gas bubbler 418. The higher gas pressure in combination with the flow of N2 further facilitate the movement of the gas of interest to the metal box 420.

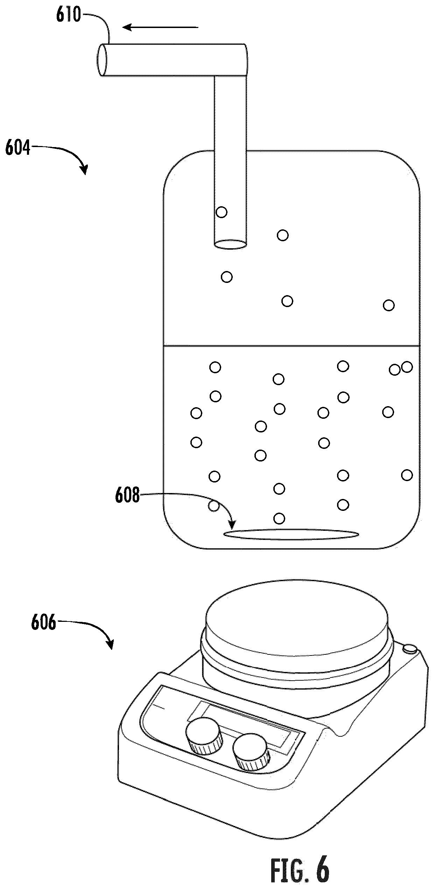

In various embodiments, the sensor test system 400 includes a water bubbler 402 and a heater 404 configured to generate humidity for varying and/or setting the humidity inside the metal box 420. The water bubbler is further described with respect to the FIG. 6 below.

In various embodiments, the water bubbler 402 generates water vapor. The second flow controller 408 controls a flow rate of the water vapor delivered to the metal box 420. The second flow controller 408 may increase a flow rate of the water vapor to increase a humidity inside the metal box 420. The second flow controller 408 may decrease a flow rate of the water vapor to decrease the humidity inside the metal box 420. In various embodiments, the controlled water vapor generated by the water bubbler 402 and second flow controller 408 is delivered to the metal box 420 using second inlet 436.

As previously described, in various embodiments, the N2 generated by the dry nitrogen gas cylinder 406 is also passed through a third flow controller 410 to generate a controlled N2 flow. The controlled N2 flow is passed to the metal box 420 using third inlet 434. By delivering more N2 to the metal box 420, a humidity inside the metal box 420 may decrease.

In various embodiments, the humidity inside the metal box 420 is measured using the humidity sensor 424. To achieve the desired humidity inside the metal box 420, a flow rate of water vapor through the second inlet 436 and a flow rate of N2 through the third inlet 434 may be adjusted. For example, if the humidity sensor 424 indicates that the humidity inside the metal box 420 is above the desired humidity level, the second flow controller 408 may decrease a flow of water vapor to the metal box 420 and/or the third flow controller 410 may increase a flow of N2 to the metal box 420. For example, if the humidity sensor 424 indicates that the humidity inside the metal box 420 is below the desired humidity level, the second flow controller 408 may increase a flow of water vapor to the metal box 420 and/or the third flow controller 410 may decrease a flow of N2 to the metal box 420. In various embodiments, the water vapor and/or the N2 also exit the metal box 420 via the first outlet 440.

In various embodiments, before starting to calibrate and/or characterize a new gas sensor or the same gas sensor 422 with a different temperature and/or humidity condition, the gas need to be removed from the metal box 420 to test and/or record how the second outlet 442 functions without the gas of interest in the metal box 420. In various embodiments, a flow of N2 from dry nitrogen gas cylinder 406 via third inlet 434 is used to purge the gas of interest in metal box 420. In various conditions, the flow of N2 from third inlet 434 may remove some of the gas, however, molecular weight of some gases, such as PC, are higher compared to N2 and the gas molecules may settle at the bottom of the metal box 420 while the N2 molecules may occupy and flow at the upper space of the metal box 420. This may cause reduce effectiveness of purging the gas using only N2.

In various embodiments, the sensor test system 400 includes a vacuum pump 428 mechanically coupled to a second outlet 442. The vacuum pump 428 may be a mechanical device used to remove gas molecules from the metal box 420 by creating a vacuum or low-pressure environment. The vacuum pump 428 may operate by reducing the pressure inside the metal box 420 below atmospheric pressure, causing molecules of the gas of interest or the substance of interest in various forms to be expelled.

In various embodiments, the vacuum pump 428 is set to a desired vacuum level, considering the pressure of the gas of interest, such as PC, inside metal box 420. For example, the pressure inside the vacuum may be set to a pressure below the pressure of the gas of interest inside the metal box 420. The vacuum pump 428 then creates a low-pressure environment inside the metal box 420, causing the gas to be drawn toward the pump. The gas may then be released to the environment after being cleansed using the scrubber. In various embodiments, the vacuum pump 428 runs for a sufficient period of time until the desired level of purging has occurred.

During the purging of metal box 420 the flow of the gas of interest through first inlet 432 is stopped. In various embodiments, the liquid gas bubbler 418 is deactivated during the purging of the gas of interest from metal box 420. The liquid gas bubbler 418 may be deactivated by stopping the flow of N2 by the first flow controller 412. In various embodiments, during the purging of metal box 420 the flow of water vapor to second inlet 436 may also be stopped, for example by stopping the flow with second flow controller 408 or by turning off heater 404.

In example embodiments, using sensor test system 400, may allow for assessing the response time, stability, and/or various other characteristics of gas sensors to determine their various performances. The gas sensor characterization tests may provide a controlled environment for calibrating gas sensors against known gas concentrations, to ensure accurate and reliable measurements during operation. Gas sensor characterization tests may also support R&D activities, enabling the study of gas sensor behaviors under various conditions, such as various pressure, temperature, and/or humidity conditions, to refine designs and develop new technologies to detect the desired gases for various purposes such as preventing thermal runaway in battery packs used in electrical vehicles, etc.

In example embodiments, the sensor test system 400 may be used for quality control. For example, the gas sensor characterization tests may help in polymer sensor quality control by identifying defective gas sensors and ensuring only those meeting performance criteria are released and/or installed.

In various embodiments, the test system is used to test the ability of one or more gas sensor to sense a presence of the gas of interest or calibrate and/or characterize the one or more sensors for sensing the gas of interest.

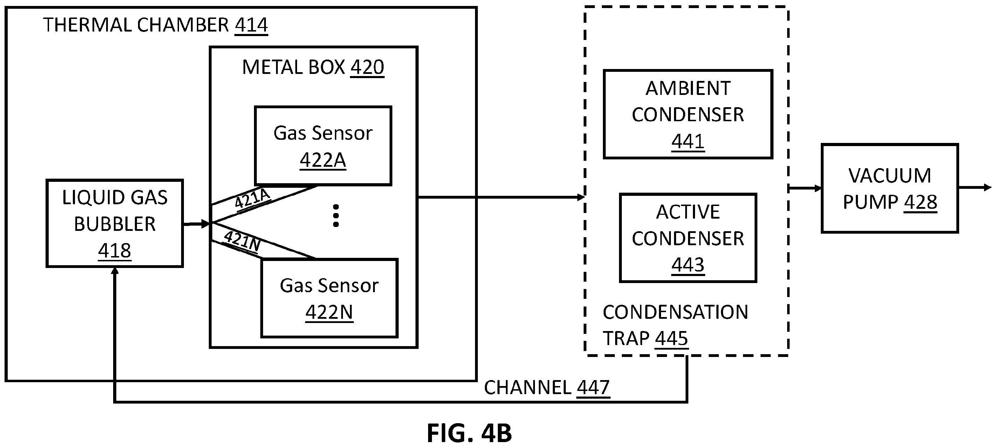

Referring now to FIG. 4B a schematic diagram illustrating various aspects of a sensor test system is provided in accordance with various embodiments of the present disclosure.

When the gas of interest enters the metal box 420, due to the weight of the gas of interest, it may settle at a lower part of the metal box. In various embodiments, a gas channel may direct the gas of interest to the gas sensor in the metal box. In various embodiments, the gas channel may be for example be a nozzle, a funnel, a pipe, etc.

In various embodiments, the metal box includes one or more gas sensors. For example, the metal box may include gas sensor 422A to gas sensor 422N. In various embodiments, any integer number of gas sensors may be used. In various embodiments, one or more gas channels are used to direct the gas of interest to the one or more gas sensors in the metal box.

In various embodiments, a single gas channel may direct the gas of interest to one or more gas sensors. In various embodiments, a corresponding gas channel may direct the gas of interest to each of the gas sensors. For example, a gas channel 421A may direct the gas of interest to a first gas sensor 422A and a gas channel 421N may direct the gas of interest to the gas sensor 422N. In various embodiments, N is an integer number greater or equal to 1. In various embodiments, the metal box 420 includes N gas sensors and N corresponding gas channels. Each gas channel may guide the gas of interest to or to the vicinity of one or more corresponding gas sensors. In various embodiments, each gas channel guides the gas of interest to a corresponding area in the metal box, where one or more gas sensors may be placed in the corresponding area.

In various embodiments, when using the test system to test the ability of the gas sensors to determine a presence of the gas of interest or to calibrate the sensor, depending on the location of the sensor in the metal box, the gas of interest may reach the gas sensor at various times or with various time delays. Testing the gas sensor may include determining a time delay of the sensor in detecting the gas of interest. To remove the delay in reaching the gas of interest from when it enters the metal box to when it reaches the gas sensor, various embodiments use the one or more gas channels such that the gas of interest reaches all the sensors at almost similar times and with a low time delay.

In various embodiments, when the gas of interest exits the thermal chamber towards the vacuum pump 428, it may pass through a condensation trap 445. The condensation trap 445 may be configured to condense some or all of the gas of interest exiting the thermal chamber to the liquid form of the gas of interest and before it reaches the vacuum pump 428. After the gas of interest is condensed to the liquid form, it may be directed back to the liquid gas bubble to be reused. Doing so, may remove or reduce the amount of the gas of interest reaching the vacuum pump 428 or is released to the environment.

In various embodiments, the condensation of the gas of interest in the condensation trap 445 may occur due to the temperature difference between the thermal chamber and the outside environment. For example, condensation trap may include ambient condenser 441 which is configured to condense the gas of interest using the ambient temperature. The ambient condenser 441 may include channels such as tubes, gas passages, etc., that are in ambient temperature.

In various embodiments, the condensation trap 445 may include an active condenser 443. In various embodiments, the active condenser condenses the gas of interest as it exits the thermal chamber using active cooling techniques. For example, the active condenser 443 may run water, or a coolant with lower temperature than the condensation temperature of the gas of interest, though the gas of interest using one or more channels or pipes.

In various embodiments, the condensation trap may include or use either of the ambient condenser 441 or the active condenser 443 to condense the gas of interest. In various embodiments, the condensation trap may include and use both of the ambient condenser 441 or the active condenser 443 to condense the gas of interest. In various embodiments, after the liquid form of the gas of interest is generated using the condensation trap 445, it is transferred to the liquid gas bubbler using a liquid gas channel 447. In various embodiments, a pump may be used to move the liquid form of the gas of interest to the gas bubbler 418. After the liquid form of the gas of interest is transferred to the gas bubble, it may be turned into the gas forms again and directed to the metal box to be reused for testing, calibrating, and/or characterizing the gas sensor(s).

In various embodiments, the condensation trap 445 passes the carrier gas and/or the water vapor to the vacuum pump. In various embodiments, by generating a low pressure, the vacuum pump facilitates flow of the gas of interest, carrier gas, and/or water vapor outside the metal chamber through the second outlet 442, however some and/or all of the gas of interest may turn into the liquid form of the gas of interest and directed to the gas bubbler using the liquid channel 447 and not reach the vacuum pump.

Referring now to FIG. 5 a schematic diagram illustrating liquid gas bubbler 500 is provided in accordance with various embodiments of the present disclosure. In various embodiments, the liquid gas bubbler 500 may include a liquid gas bubbler inlet 504 where the carrier gas (e.g., N2) is entered. In various embodiments, the carrier gas may be any other inert gas such as N2 may then pass through a cap 508 to break the large bubbles into finer bubbles in a container 510 that holds a liquid form of the substance of interest such as liquid PC. In various embodiments, the cap 508 is a sintered tube. When N2 passes through the liquid form of the gas, and bubbles are formed, and the substance of interest is released in the gas form to generate the gas of interest even at temperatures below its boiling point.

In example embodiments, using the cap 508 increases the liquid-N2 interface area and promotes the transfer of liquid molecules into the gas form. N2 may also pick up gas molecules from the liquid surface and carries them away through the liquid gas bubbler outlet 506, resulting in the evaporation of liquid into the gas. In various embodiments, by placing liquid gas bubbler 500 in the thermal chamber 414 and increasing its temperature, turning the liquid form of the substance of interest to its gas form is further facilitated.

Referring now to FIG. 6 a schematic diagram illustrating water bubbler 604 and heater 606 is provided in accordance with various embodiments of the present disclosure. In various embodiments, the water bubbler 604 includes a coin 608 immersed in water. The heater 606 may be a magnetic and/or an inductive heater. The heater 606 may heat the water and turn it into water vapor. A magnetic heater 606 may cause the coin 608 to rotate at high-speed creating water vapor that exits from outlet 610. The heater 606 may cause the coin 608 to turn at the high speed and may increase its temperature to turn the water-to-water vapor at a high rate. The water vapor exits at the outlet 610, its flow is controlled using second flow controller 408 before it enters metal box 420 via second inlet 436.

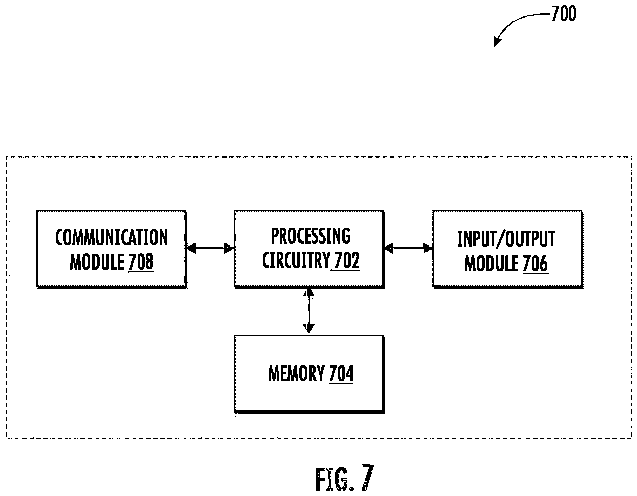

Referring now to FIG. 7, a schematic diagram depicting an example controller 700 of an example apparatus in electronic communication with various other components in accordance with various embodiments of the present disclosure is provided. For example, referring to FIG. 4A, as described herein the controller 700 may be in electronic communications with and/or control any of the components of the sensor test system 400 such as at least one of the second flow controller 408, third flow controller 410, and/or first flow controller 412, heater 404, temperature sensor 426, humidity sensor 424, gas sensor 422, and/or vacuum pump 428. As shown, the controller 700 comprises processing circuitry 702, a communication module 708, input/output module 706, a memory 704 and/or other components configured to perform various operations, procedures, functions or the like described herein.

The processing circuitry 702 may be implemented as, for example, various devices comprising one or a plurality of microprocessors with accompanying digital signal processors; one or a plurality of processors without accompanying digital signal processors; one or a plurality of coprocessors; one or a plurality of multi-core processors; one or a plurality of controllers; processing circuits; one or a plurality of computers; and various other processing elements (including integrated circuits, such as ASICs or FPGAs, or a certain combination thereof). In some embodiments, the processing circuitry 702 may comprise one or more processors. In one exemplary embodiment, the processing circuitry 702 is configured to execute instructions stored in the memory 504 or otherwise accessible by the processing circuitry 702. When executed by the processing circuitry 502, these instructions may enable the controller 700 to execute one or a plurality of the functions as described herein. No matter whether it is configured by hardware, firmware/software methods, or a combination thereof, the processing circuitry 702 may comprise entities capable of executing operations according to the embodiments of the present invention when correspondingly configured. Therefore, for example, when the processing circuitry 702 is implemented as an ASIC, an FPGA, or the like, the processing circuitry 702 may comprise specially configured hardware for implementing one or a plurality of operations described herein. Alternatively, as another example, when the processing circuitry 702 is implemented as an actuator of instructions (such as those that may be stored in the memory 704), the instructions may specifically configure the processing circuitry 702 to execute one or a plurality of methods, algorithms and operations described herein, such as those discussed with reference to any of the figures herein.

The memory 704 may comprise, for example, a volatile memory, a non-volatile memory, or a certain combination thereof. Although illustrated as a single memory in FIG. 7, the memory 704 may comprise a plurality of memory components. In various embodiments, the memory 704 may comprise, for example, a hard disk drive, a random-access memory, a cache memory, a flash memory, a Compact Disc Read-Only Memory (CD-ROM), a Digital Versatile Disk Read-Only Memory (DVD-ROM), an optical disk, a circuit configured to store information, or a certain combination thereof. The memory 704 may be configured to store information, data, application programs, instructions, and etc., so that the controller 700 can execute various functions according to the embodiments of the present disclosure. For example, in at least some embodiments, the memory 704 is configured to cache input data for processing by the processing circuitry 702. Additionally, or alternatively, in at least some embodiments, the memory 704 is configured to store program instructions for execution by the processing circuitry 702. The memory 704 may store information in the form of static and/or dynamic information. When the functions are executed, the stored information may be stored and/or used by the controller 700.

The communication module 708 may be implemented as any apparatus included in a circuit, hardware, a computer program product or a combination thereof, which is configured to receive and/or transmit data from/to another component or apparatus. The computer program product comprises computer-readable program instructions stored on a computer-readable medium (for example, the memory 704) and executed by a controller 700 (for example, the processing circuitry 702). In some embodiments, the communication module 708 (as with other components discussed herein) may be at least partially implemented as the processing circuitry 702 or otherwise controlled by the processing circuitry 702. In this regard, the communication module 708 may communicate with the processing circuitry 702, for example, through a bus. The communication module 708 may comprise, for example, antennas, transmitters, receivers, transceivers, network interface cards and/or supporting hardware and/or firmware/software and is used for establishing communication with another apparatus. The communication module 708 may be configured to receive and/or transmit any data that may be stored by the memory 704 by using any protocol that can be used for communication between apparatuses. The communication module 708 may additionally or alternatively communicate with the memory 704, the input/output module 706 and/or any other component of the controller 700, for example, through a bus.

In some embodiments, the controller 700 may comprise an input/output module 706. The input/output module 706 may communicate with the processing circuitry 702 to receive instructions input by the user and/or to provide audible, visual, mechanical, or other outputs to the user. Therefore, the input/output module 706 may be in electronic communication with supporting devices, such as a keyboard, a mouse, a display, a touch screen display, and/or other input/output mechanisms. Alternatively, at least some aspects of the input/output module 706 may be implemented on a device used by the user to communicate with the controller 700. The input/output module 706 may communicate with the memory 704, the communication module 708 and/or any other component, for example, through a bus. One or a plurality of input/output modules and/or other components may be included in the controller 700.

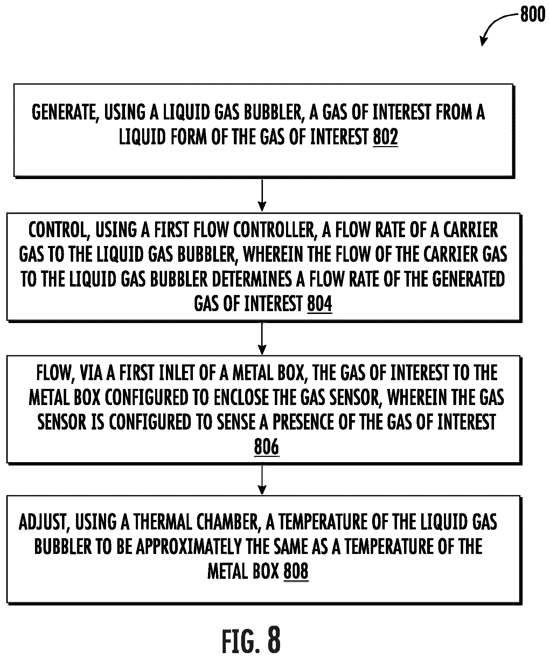

Referring now to FIG. 8, a flowchart diagrams illustrating example method 800, in accordance with various embodiments of the present disclosure are provided. Referring to FIG. 7, in some examples, various steps of the method 800 may be performed by a control system such as one using controller 700. Various aspects of the methods are described with reference to the sensor test system 400 described with reference to FIG. 4A, FIG. 4B, FIG. 5, or FIG. 6.

In various embodiments, in block 802, method 800 generates, using a liquid gas bubbler 418, a gas of interest from a liquid form of the gas of interest. For example, PC gas may be formed from liquid PC using the liquid gas bubbler described with reference to FIG. 4A, FIG. 4B, and FIG. 5.

In various embodiments, in block 804, method 800 controls, using a first flow controller 412, a flow rate of a carrier gas (for example N2) to the liquid gas bubbler 418. The flow of the carrier gas to the liquid gas bubbler 418 may determine a flow rate of the generated gas of interest. For example, by increasing a flow of N2 to the liquid gas bubbler 418, a higher flow rate of the gas of interest to the metal box 420 is generated.

In various embodiments, in block 806, method 800 flows the gas of interest, via a first inlet 432 of a metal box 420. The metal box 420 may be configured to enclose the gas sensor 422. The gas sensor 422 may be configured to sense a presence of the gas of interest in the metal box 420.

In various embodiments, in block 808, method 800 adjusts, using a thermal chamber 414, a temperature of the liquid gas bubbler 418 to be approximately the same as a temperature of the metal box 420. In various embodiments, the thermal chamber 414 is configured to enclose the liquid gas bubbler 418 and the metal box 420.

In various embodiments, the method 800 may flow the carrier gas to the metal box, via a second inlet 436 of the metal box 420. The method 800 may control, via a second flow controller 408, a flow rate of the carrier gas to the metal box 420. In various embodiments, the second flow controller 408 is placed between a carrier gas container, such as dry nitrogen gas cylinder 406, and the metal box 420.

In various embodiments, the method 800 controls a concentration of the gas of interest in the metal box 420 by controlling, using the first flow controller 412, the flow rate of the gas of interest to the metal box, and controlling, using the second flow controller 408, the flow rate of the carrier gas to the metal box.

In various embodiments, the method 800 generates water vapor using a water bubbler 402, flows the water vapor to the metal box using a third inlet 434 of the metal box, and controls a flow rate of water vapor to metal box 420 using a third flow controller 410. In various embodiments, the third flow controller is placed between the water bubbler and the metal box.

In various embodiments, method 800 heats a metal coin in water contained in the water bubbler using a magnetic heater. In various embodiments, the method 800 heats the water in the water bubbler using the heated metal coin. In various embodiments, method 800 rotates the metal coin in the water contained in the water bubbler to generate water vapor using the magnetic heater.

In various embodiments, method 800 controls a humidity inside the metal box by controlling, using the second flow controller, the flow rate of the carrier gas to the metal box, and controlling, using the third flow controller, the flow rate of the water vapor to the metal box.

In various embodiments, method 800 measuring a humidity inside the metal box using a humidity sensor in the metal box and controlling the humidity inside the metal box to a desired humidity level using the measured humidity inside the metal box. For example, if the measured humidity is below a desired level, method 800 increases the humidity in metal box 420, and if the humidity is below the desired level, method 800 reduces the humidity inside the metal box 420.

In various embodiments, method 800 measures, using a temperature sensor in the metal box, a temperature inside the metal box, and controls the temperature inside the metal box to a desired temperature level using the measured temperature inside the metal box.

With reference to FIG. 4A, FIG. 4B, FIG. 5, FIG. 6, FIG. 7, in various embodiments, a method converts, using a liquid gas bubbler 418 and a carrier gas, a substance of interest from a liquid form to a gas form. The method may flow the gas from of the substance of interest to a metal box 420 via a first inlet 432 of the metal box 420. In various embodiments, the method may vary a humidity level inside the metal box 420 by flowing water vapor generated by a water bubbler 402 to the metal box 420 using a second inlet 436 of the metal box 420.

In various embodiments, the method may flow the carrier gas directly to the metal box 420 using a third inlet 434 of the metal box 420. The method may control a flow rate of the gas form of the substance of interest, a flow rate of water vapor, and a flow rate of the carrier gas to the metal box 208 as described herein. In various embodiments, the method may control a temperature of the liquid gas bubbler 418 and the metal box 420 using a thermal chamber 414 enclosing the liquid gas bubbler 418 and the metal box 420. In various embodiments, the method may purge the substance of interest from the metal box 420 using the carrier gas and a vacuum pump.

Many modifications and other embodiments of the present disclosure set forth herein will come to mind to one skilled in the art to which these embodiments pertain having the benefit of the teachings presented in the foregoing descriptions and the associated drawings. Therefore, it is to be understood that the disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A sensor test system comprising:
a metal box comprising a first inlet, wherein the metal box is configured to enclose a gas sensor configured to sense a presence of a gas of interest;
a liquid gas bubbler configured to receive a carrier gas and generate the gas of interest from a liquid form of the gas of interest using the carrier gas;
a first flow controller configured to control a flow rate of the carrier gas to the liquid gas bubbler and the gas of interest from the liquid gas bubbler to the first inlet of the metal box; and
a thermal chamber configured to enclose the liquid gas bubbler and the metal box and configured to set a temperature inside the thermal chamber to a desired temperature.

2. The sensor test system of claim 1, further comprising:
a gas channel configured to direct the gas of interest from the first inlet of the metal box to the gas sensor;
a carrier gas container configured to:
provide the carrier gas to the liquid gas bubbler, wherein the first flow controller is placed between the carrier gas container and the liquid gas bubbler; and
provide carrier gas to the metal box through a second inlet of the metal box; and
a second flow controller placed between the carrier gas container and the second inlet of the metal box and configured to control a flow rate of the carrier gas to the metal box.

3. The sensor test system of claim 2, further comprising:
a water bubbler configured to provide water vapor to the metal box through a third inlet of the metal box; and
a third flow controller placed between the water bubbler and the third inlet and configured to control a flow rate of water vapor to the metal box.

4. The sensor test system of claim 3, further comprising a heater configured to increase a temperature of water in the water bubbler and facilitate generating the water vapor.

5. The sensor test system of claim 4, wherein:
the water bubbler comprises a metal coin configured to rotate at a high speed and to cause the water to vaporize; and
the heater is a magnetic heater and is configured to cause the coin to rotate and increase a temperature of the coin to facilitate generating the water vapor.

6. The sensor test system of claim 3, further comprising:
a vacuum pump coupled to a second outlet of the metal box, wherein the vacuum pump configured to purge the gas of interest from the metal box in combination with a flow of the carrier gas through the metal box; and
a condensation trap comprising an ambient condenser or an active condenser, wherein the condensation trap is coupled to the second outlet of the metal box and placed outside the thermal chamber and is configured to convert the gas of interest to the liquid form of the gas of interest; and
a liquid gas channel coupled to the condensation trap and the liquid gas bubbler, the liquid gas channel configured to direct the liquid form of the gas of interest to the gas bubbler.

7. The sensor test system of claim 2, wherein the liquid gas bubbler comprises a sintered tube configured to break large bubbles generated by the carrier gas to finer bubbles and facilitate generating the gas of interest.

8. A method for testing a gas sensor, the method comprising:
generating, using a liquid gas bubbler, a gas of interest from a liquid form of the gas of interest;
controlling, using a first flow controller, a flow rate of a carrier gas to the liquid gas bubbler, wherein the flow of the carrier gas to the liquid gas bubbler determines a flow rate of the generated gas of interest;
flowing, via a first inlet of a metal box, the gas of interest to the metal box configured to enclose the gas sensor, wherein the gas sensor is configured to sense a presence of the gas of interest; and
adjusting, using a thermal chamber, a temperature of the liquid gas bubbler to be approximately the same as a temperature of the metal box.

9. The method of claim 8, further comprising:
converting, using a condensation trap comprising an ambient condenser or an active condenser placed outside the thermal chamber, the gas of interest to the liquid form of the gas of interest; and
directing, using a liquid channel coupled to the condensation trap and the liquid gas bubbler, the liquid form of the gas of interest to the gas bubbler,
wherein the thermal chamber is configured to enclose the liquid gas bubbler and the metal box.

10. The method of claim 8, further comprising:
directing, via a gas channel, the gas of interest to the gas sensor from the first inlet of the metal box;
flowing, via a second inlet of the metal box, the carrier gas to the metal box; and
controlling, via a second flow controller, a flow rate of the carrier gas to the metal box, wherein the second flow controller is placed between a carrier gas container and the metal box.

11. The method of claim 10, further comprising controlling a concentration of the gas of interest in the metal box by:
controlling, using the first flow controller, the flow rate of the gas of interest to the metal box; and
controlling, using the second flow controller, the flow rate of the carrier gas to the metal box.

12. The method of claim 10, further comprising:
generating, using a water bubbler, water vapor;
flowing, using a third inlet of the metal box, the water vapor to the metal box; and
controlling, using a third flow controller, a flow rate of water vapor, wherein the third flow controller is placed between the water bubbler and the metal box.

13. The method of claim 12, further comprising:
heating, using a magnetic heater, a metal coin in water contained in the water bubbler;
heating, using the metal coin, the water in the water bubbler; and
rotating, using the magnetic heater, the metal coin in the water contained in the water bubbler to generate water vapor.

14. The method of claim 12, further comprising controlling a humidity inside the metal box by:
controlling, using the second flow controller, the flow rate of the carrier gas to the metal box; and
controlling, using the third flow controller, the flow rate of the water vapor to the metal box.

15. A method comprising:
converting, using a liquid gas bubbler and a carrier gas, a substance of interest from a liquid form to a gas form;
flowing the gas from of the substance of interest to a metal box via a first inlet of the metal box;
varying a humidity level inside the metal box by flowing water vapor generated by a water bubbler to the metal box using a second inlet of the metal box;
flowing the carrier gas directly to the metal box using a third inlet of the metal box;
controlling a flow rate of the gas form of the substance of interest, a flow rate of water vapor, and a flow rate of the carrier gas to the metal box;
controlling a temperature of the liquid gas bubbler and the metal box using a thermal chamber enclosing the liquid gas bubbler and the metal box; and
purging the substance of interest from the metal box using the carrier gas and a vacuum pump.
